# EUROPEAN PATENT APPLICATION

(11) **EP 2 845 852 A1**
(43) Date of publication of application: **11.03.2015**
(21) Application number: 14183556.1
(22) Date of filing: 04.09.2014
(51) Int. Cl.: C07D 223/26, C07D 257/02, C07D 487/22, C06B 25/34

(54) **A method to produce and scale-up cocrystals and salts via resonant acoustic mixing**

(30) Priority: 04.09.2013 US 201361873656 P; 28.08.2014 US 201414471593
(71) Applicant: Nalas Engineering Services Inc., Centerbrook, CT 06409 (US)
(72) Inventor: Salan, Jerry, Salem, CT Connecticut 06420 (US); Anderson, Stephen R., Stonington, CT Connecticut 06378 (US); am Ende, David J., East Lyme, CT Connecticut 06333 (US)
(74) Representative: Klunker . Schmitt-Nilson . Hirsch

(57) **Abstract**

A method to produce and manufacture cocrystals and salts is disclosed wherein crystalline solids and other components were combined in the desired proportions into a mixing chamber and mixed at high intensity to afford a cocrystalline product. No grinding media were required. The mixing system consists of a resonant acoustic vibratory system capable of supplying a large amount of energy to the mixture and is tunable to a desired resonance frequency and amplitude. The use of resonant acoustic mixing to assist cocrystallization is novel. This discovery enables the manufacture of cocrystals and salt forms, simplifying their manufacture and scale-up, and avoiding the use of grinding methods or grinding media. The present invention affords the manufacture of cocrystals and salts on kilogram to multi-ton scale and is adaptable to continuous manufacturing through the use of resonant mixing methods. (Drawing - Fig. 2)

## Description

### CROSS REFERENCE TO RELATED APPLICATION(S)

This application claims the benefit of United States Provisional Patent Application No. 61/873,656, which was filed September 4, 2013. The disclosure of application serial number 61/873,656 is incorporated by reference herein in its entirety.

### UNITED STATES GOVERNMENT RIGHTS

None.

### BACKGROUND

Cocrystals can be thought of simply as a multi-component crystal. More specifically a cocrystal can be understood as a multi-component molecular crystal in a defined stoichiometric ratio whose components are non-covalently bonded via hydrogen bonding or other van der Waals interactions. A salt is usually thought of as a multi-component solid containing minimally ionic bonds between an acid and base. A cocrystal is a crystalline molecular complex which may include an ionic pair among the different components and by definition includes solvates (hydrates), inclusion compounds, clathrates and solid solutions (Reutzel-Edens, Susan M. Analytical Techniques and Strategies for Salt/Cocrystal Characterization, Fundamental Aspects of Salts and Cocrystals, in Pharmaceutical Salts and Co-crystals, Edited by Johan Wouters and Luc Quere, RSC Drug Discovery Publishing 2012, incorporated herein by reference in its entirety). Thus cocrystal hydrates and solvates as well as salts and their respective hydrates and solvates are within scope of this disclosure. An important distinction between salts and cocrystals is that salts are necessarily made up of acid and bases, cocrystals are not. Thus cocrystals expand the options for altering solid-state properties beyond the pKa criteria required for salt-forms. Collectively cocrystals and salts provide a wide array of options to engineer viable solid-state forms.

Cocrystals are well known to industry especially in the pharmaceutical industry and the energetics community. Cocrystals are important to the pharmaceutical industry because they provide an opportunity for tuning the physicochemical properties of active pharmaceutical ingredients (APIs). For example, cocrystals may be used to attenuate solubility, dissolution properties, hygroscopicity, mechanical properties, particle size, thermal properties, stability, and enhance bioavailability of a poorly soluble drug or simply improve chemical stability characteristics. In some embodiments, as used herein a pharmaceutical compound is a compound intended for use in the diagnosis, cure, mitigation, treatment, or prevention of disease or as a component of a medicine used to diagnosis, cure, mitigate, treat, or prevent a disease. In some embodiments, the term pharmaceutical compound includes an API, the active or central ingredient which causes the direct effect on the disease diagnosis, prevention, treatment or cure. In some embodiments, the term pharmaceutical compound includes an excipient, an inactive or inert substance present in a drug product.

Similarly, in energetics applications, cocrystals provide an opportunity to tailor performance characteristics of explosives and insensitive munition (IM's). For example, to ensure safe application of insensitive munitions the energetic material must be formulated or prepared in such a way to reduce sensitivity to accidental initiation. Cocrystals afford an opportunity to tune the performance characteristics as well as the sensitivity characteristics of energetic materials. In some embodiments, as used herein an energetic compound is any material such as an explosive, propellant, or pyrotechnic that can attain a highly energetic state such as by chemical reactions. In some embodiments, the energetic compounds contemplated for use in the present disclosure include, but are in no way limited to CL20 (hexanitrohexazaisowurtzetane), RDX (cyclo-1,3,5-trimethylene-2,4,6-trinitramine), Fox-7 (1,1-diamino-2,2-dinitroethene), HMX (cyclotetramethylenetetranitramine), ADN (ammonium dinitramide), TNT (2,4,6-trinitrotoluene), NTO (3-nitro-1,2,4,-triazol-5-one), ammonium nitrate, and ammonium perchlorate. Although cocrystals and salts find application primarily in pharmaceuticals and energetics fields, they are not limited to these areas.

One of the main challenges that exist with cocrystallization methods is their ability to scale-up to multi-kilogram scale. Common laboratory methods in current practice are mortar-pestle grinding and ball milling, neither of which are amenable to large scale manufacturing. Current methods of forming cocrystals at lab scale include direct solution phase crystallization, solid-state grinding, liquid assisted grinding, slurry conversion, and ultrasonic assisted methods (Sachit Goyal, Michael R. Thorson, Geoff G. Z. Zhang, Yuchuan Gong, and Paul J. A. Kenis. Microfluidic Approach to Cocrystal Screening of Pharmaceutical Parent Compounds. Cocrystal Growth & Design. October 22, 2012, incorporated by reference herein in its entirety).

Although solution phase crystallization and slurry conversions are amenable to scale-up to stirred tanks, the processes are complicated to design due to the solubility constraints of each coformer and the kinetics of the crystallization systems. For example the design of solution phase cocrystallization process requires:
1) Identification of an appropriate solvent system that meet the solubility requirements;
2) Understanding multicomponent solid-liquid phase equilibria and identification of a path forward; and
3) A method to induce nucleation (e.g. seeding) and control supersaturation (e.g. cooling and the like).

Given the dual solubility constraints of each coformer, a solution cocrystallization process may not be feasible or even possible for certain cocrystals combinations. Depending on the phase equilibria, solvates of either coformer may form instead. Therefore traditional solution crystallization provides only a narrow window of scope for scale-up of cocrystallization process.

Solid-state grinding to induce a chemical change (aka mechanochemistry) offers advantages because it can be used to produce cocrystals that are unobtainable by other methods. Solid state grinding, either dry or with a small amount of solvent added (liquid-assisted grinding) is typically achieved through mortar and pestle grinding or ball-mill grinding where grinding media are employed. Grinding media include stainless steel balls or ceramic media. Solid-state grinding approaches are difficult to scale to manufacturing multi-kilogram or larger quantities. For energetic materials solid-state grinding with grinding media presents a potential hazard due to friction generated during grinding, potentially initiating an energetic material resulting in detonation or deflagration. Ultrasonics of powders have issues creating homogeneity across the sample or mixture and are also not readily scaled for powder systems.

The current disclosure combines the concept of solid-state grinding (neat and liquid assisted) with high intensity resonant mixing resulting in a general yet scalable method for manufacturing cocrystals and salts. Furthermore, experiments have shown that cocrystallization experiments performed under resonant acoustic mixing resulted in higher success rate of forming cocrystals as compared to liquid assisted grinding method (discussed below in specific examples). The resonant mixing method to form cocrystals is more general than the current solid state grinding, liquid-assisted grinding, or solution phase cocrystallization approaches. In fact the conditions of the resonant mixing method leverage the part of the phase diagram where the relative solubilities of the coformers are no longer relevant because the amount of liquid is small enough to keep it fully saturated with respect to all components (Friscic, Tomislav, and W. Jones, Application of Mechanochemistry in the Synthesis and Discovery of New Pharmaceutical Forms: Cocrystal, Salts, and Coordination Compounds, Fundamental Aspects of Salts and Cocrystals, in Pharmaceutical Salts and Co-crystals, Edited by Johan Wouters and Luc Quere, RSC Drug Discovery Publishing 2012, incorporated herein by reference in its entirety). The present disclosure is also more generally conducive to scale-up and amenable to current good manufacturing practices (cGMP manufacturing) than current ball mill methods. Resonant mixers are available at both lab and manufacturing scale.

Polymorphism is defined as the ability of a substance to exist as two or more crystalline phases that have different arrangements and/or conformations of the molecules in the crystal lattice (Grant, David J. W., Theory and Origin of Polymorphism, in Polymorphism in Pharmaceutical Solids, edited by Harry G. Brittain, Marcel-Dekker, 1999, incorporated herein by reference in its entirety). Polymorphic solids have a different molecular packing arrangement stemming from variation in hydrogen bonding and/or van der Waals interactions, and hence display different physical properties. Trask et. al. has shown that solid-state grinding methods in the presence of a small amount of solvent can induce specific polymorphic transformations (Trask, Andrew V., and W. Jones, Top. Curr Chem (2005) 254:41-70, incorporated herein by reference in its entirety). Given the present discovery that acoustic resonance mixing is an effective mechanochemical method for generating cocrystals, by extension therefore acoustic resonant mixing should be conducive to polymorphic transformations as well and is so claimed.

The disclosure of US 2013/0164664 A1, incorporated herein by reference in its entirety, provides substantial background on resonant acoustic mixing.

The disclosure of US 2008/0038557 A1, incorporated herein by reference in its entirety, suggests acoustic mixers provided by RESODYN Acoustic Mixers, Inc. as preferred vibratory mixers.

The disclosure of US 2013/0102781 A1, incorporated herein by reference in its entirety, provides background for the solution and slurry method of cocrystallation.

The disclosure of WO2006007448A2, incorporated herein by reference in its entirety, describes a cocrystallized compound incorporating an API, as well as methods of making and using the same.

### SUMMARY

The present disclosure is ideal for energetics and pharmaceutical cocrystal applications. Applications of resonant- acoustic mixing assisted cocrystallizations for both energetic and pharmaceutical cocrystals are being pursued. For energetic cocrystals, the disclosure offers a safer (no grinding media involved) option for producing cocrystals. It also offers a greener (less solvent), higher yielding, and more convenient option to solution phase cocrystallizations. The present disclosure also widens the scope of potential cocrystals for pharmaceutical application. Resonant-acoustic mixing assisted cocrystallization results in a higher success rate of cocrystal formation than liquid assisted grinding or solution phase crystallizations. The resonant acoustic mixing platform as exemplified is an ideal platform for cocrystal screening to find new cocrystal forms. In addition the present disclosure provides a scale-up technology to produce cocrystals on kilogram and multi-kilogram scale.

In one embodiment, the present disclosure is directed to a process for making a cocrystal from two or more compounds by combining compounds in a suitable vessel and subjecting the vessel and mixture to resonant acoustic mixing. In another embodiment, the process of the present disclosure is performed in a resonant acoustic mixer. In a further embodiment, the resonant-acoustic mixer used in the examples described herein is sold under the trademark LABRAM^{®} and available from RESODYN. In one embodiment, at least one of the compounds is a coformer. In other embodiments, at least one of the compounds is a pharmaceutical or energetic compound. In further embodiments, at least one of the compounds is a suitable counter-ion. In another embodiment, at least one of the compounds is a salt. In another embodiment, at least one of the compounds is a neutral non-ionic solid. In a further embodiment, at least one of the compounds is an API.

In some embodiments, a resonant frequency of mixing is from about 5 Hz to about 2000 Hz, but more preferred is 50 to 1000 Hz, and most preferred is in the range of 50 to 100 Hz. The resultant accelerations for mixing is in the range of 2 to 200 g-forces but more preferred is in the range of 10 to 150 g-forces. In some embodiments, the energy of mixing is between about 50 to 150 g-forces. In some embodiments, the intensity of mixing is between about 30 and 110 g-forces. In a further embodiment, a solvent or mixture of solvents is added. In one embodiment, the solvent is one in which one or more coformer is slightly soluble in to mediate the solid-solid interactions between coformers to form cocrystals. In some embodiments, as used herein, the terms "soluble" and "slightly soluble" are used to generally mean that a solvent provides at least some level of solubility for the respective components. In some embodiments, the terms "soluble" and "slightly soluble" mean a solubility of the compound of at least 1 part solute per 10,000 parts solvent (i.e. at least 0.01 mg/ml). In another embodiment, solvent or mixture of solvents or the presence of water vapor leads to the formation of a cocrystal hydrate or cocrystal solvate.

In one embodiment, the present disclosure is directed to a process for making a crystalline salt form by combining two compounds in a suitable vessel and subjecting the vessel and mixture to resonant acoustic mixing. In another embodiment, the present disclosure is directed to a process for effecting a polymorph transformation of a compound by combining the compound in the presence of a solvent or mixture of solvents in a suitable vessel and subjecting the vessel and the mixture to resonant acoustic mixing.

In a further embodiment, the present disclosure is directed to a process for screening solid state forms such as cocrystals, salts, and polymorphs with the intention of discovering new solid state forms of similar composition, such as cocrystals or salts, or with different compositions, such as acid, base, or neutral components of cocrystals or salts including varying experimental conditions using resonant acoustic mixing. The ability of the LABRAM mixer to execute in parallel multiple small scale experiments provides a rapid and convenient method for this activity.

In one embodiment, the present disclosure further comprises increasing a volume of the resonant acoustic mixer to perform a scaled-up process for making a cocrystal from two or more compounds. In some embodiments, the present disclosure further comprises cocrystals, crystalline salts, and polymorphs made by the above-described processes.

### BRIEF DESCRIPTION OF THE DRAWINGS

The drawings show embodiments of the disclosed subject matter for the purpose of illustrating the invention. However, it should be understood that the present application is not limited to the precise arrangements and instrumentalities shown in the drawings, wherein:
FIG. 1 shows Liquid Assisted Grinding experiments to form cocrystals of carbamazepine adapted from Weyna et. al.
FIG. 2 shows cocrystallization of carbamazepine with 8 coformers in the presence of 8 solvents produced from resonance acoustic mixing.
FIG. 3 shows an x-ray powder diffraction (PXRD) pattern for 151.7 mg of carbamazepine:nicotinamide cocrystal formed in the presence of 30 µl of methanol as solvent, assisted using resonant acoustic mixing (LABRAM mixer) at 90% intensity (61 Hz and 100 g-forces) after 2 hours of mixing.
FIG. 4 shows a PXRD pattern for carbamazepine:nicotinamide (cbz:nct) formed in the presence of a range of solvents, assisted using resonant acoustic mixing (LABRAM mixer) at 90% intensity (61 Hz and 100 g-forces); 100 mg of carbamezapine (cbz) + 51.7 mg of nicotinamide (nct) were mixed with 30 microliter of solvent.
FIG. 5 shows a PXRD pattern for 151.7 mg of carbamazepine:nicotinamide (cbz:nct) formed in the presence of 30 µl of methanol as solvent, assisted using resonant acoustic mixing (LABRAM mixer) at 90% intensity (61 Hz and 100 g-forces) for varying durations.
FIG. 6 shows a PXRD pattern for carbamazepine:4,4-bipyridine cocrystals formed in the presence of solvent, assisted using resonant acoustic mixing (LABRAM mixer) at 90% intensity (61 Hz and 100 g-forces); 100 mg of carbamazepine (cbz) + 66 mg (4,4-bipyridine) were mixed with 33 µL of solvent.
FIG. 7 shows a PXRD pattern for carbamazepine:4-aminobenzoic acid cocrystals formed in the presence of solvent, assisted using resonant acoustic mixing (LABRAM mixer) at 90% intensity (61 Hz and 100 g-forces); 100 mg of carbamezapine (cbz) + 66 mg (4-aminobenzoic acid) were mixed with 32µL of solvent.
FIG. 8 shows a PXRD pattern for carbamazepine:terephthaladehyde cocrystals formed in the presence of solvent, assisted using resonant acoustic mixing (LABRAM mixer) at 90% intensity (61 Hz and 100 g-forces); 100 mg of carbamezapine (cbz) + 56.8 mg terephthaladehyde were mixed with 31 µL of solvent.
FIG. 9 shows a differential scanning calorimetry (DSC) scan of carbamazepine:nicotinamide cocrystal prepared with deionized (DI) water and resonance acoustic mixing.
FIG. 10 shows three DSC curves: a) carbamazepine :nicotinamide top: wet with dimethylformamide (DMF), middle: after drying of residual DMF, bottom: after reslurry in acetonitrile, filtration, and drying of residual acetonitrile.
FIG. 11 shows a DSC curve of carbamazepine:4-aminobenzoic acid 1:1 cocrystal produced from MeOH and resonance acoustic mixing.
FIG. 12 shows a PXRD pattern overlay of carbamazepine:nicotinamide cocrystal prepared from DMF with acetonitrile reslurry on a 1.5 gram scale (bottom curve) and 22 gram scale (top curve) by resonance acoustic mixing compared with a calculated powder pattern (middle curve) from the single crystal structure for carbamazepine :nicotinamide (I) obtained from Fleischman et. al.
FIG. 13 shows a PXRD pattern for CL-20-acetonitrile solvate form generated in a LABRAM mixer (top) overlaid with known polymorphic forms of CL20.
FIG. 14: Powder x-ray diffraction patterns for the 2CL20:HMX cocrystal produced from RAM (2:1) at 100 mg scale (middle) compared with co-crystal produced from solution 40 g scale (top) (2 to 37° 2-theta) and the calculated powder pattern from the single-crystal x-ray structure (bottom).

### DETAILED DESCRIPTION

Solvent drop grinding (SDG) is also referred to as liquid assisted grinding (LAG), wet cogrinding, mechanochemistry, or solid-state grinding by mortar and pestle or ball mill. The solid-state approach is well precedented and has been demonstrated to produce materials which, in some cases, will not form by traditional solution crystallization approaches. The present disclosure was put to practice by demonstrating that cocrystals could be formed through the use of resonant acoustic mixing. Previously published results on cocrystals formed by liquid assisted grinding approaches were used to demonstrate that cocrystallization by resonant acoustic mixing was feasible. An 8x8 matrix of 64 experiments using carbamazepine as coformer A and a coformer B from the following list of coformers: nicotinamide, 4,4 bipyridine, 4-aminobenzoic acid, 2,6 pyridine dicarboxylic acid, p-benzoquinone, terephthalaldehyde, saccharin, and aspirin was performed. In each cocrystallization experiment, a small amount of solvent was used from the following list: chloroform (CHCl₃), water, dimethylformamide (DMF), dimethylsulfoxide (DMSO), methanol (MeOH), cyclohexane, toluene, and ethyl acetate.

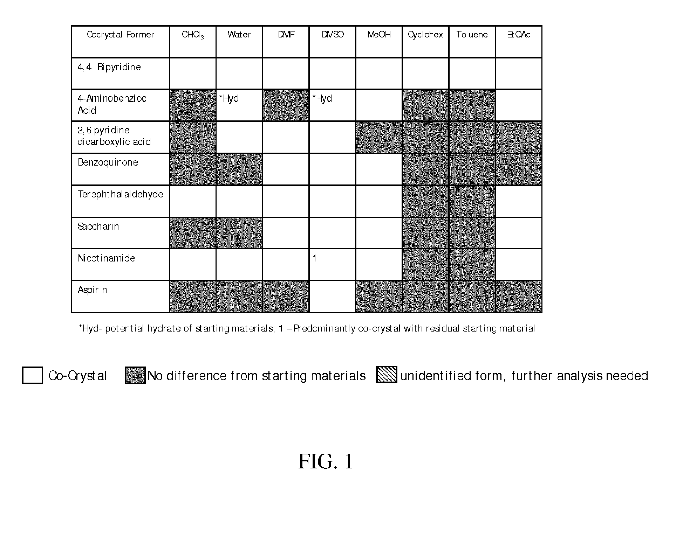

The published results were taken from Weyna et. al., Synthesis and Structural Characterization of Cocrystals and Pharmaceutical Cocrystals: Mechanochemistry vs Slow Evaporation from Solution, Crystal Growth & Design, Vol. 9, No. 2, 2009, pp 1106-1123, incorporated herein by reference in its entirety. The results of Weyna et. al. for carbamazepine (cbz) indicated a success rate of 35 out of 64 for this set of experiments or 55%. The 29 combinations that did not form cocrystals from their study simply resulted in mixtures of starting material, without significant product cocrystal being formed, as can be seen in Figure 1.

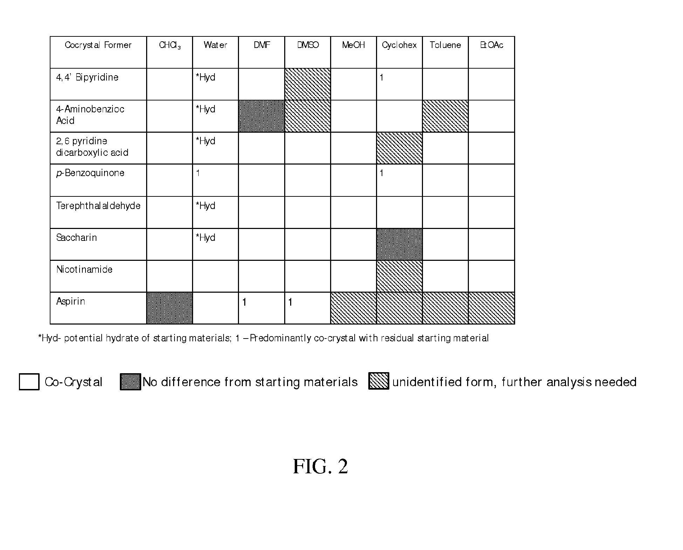

Performing the same set of 64 cocrystallization experiments in a lab-scale resonant acoustic mixer (LABRAM mixer), a success rate of 52 out of 64 cocrystallizations were obtained indicating greater than 80% success rate. There was a significantly higher success rate using resonant acoustic mixing to facilitate cocrystallization than by solvent drop grinding method (80% success rate vs 55% by solvent grinding). The higher success rate with resonant acoustic mixing was unexpected and non-obvious. The matrix of experiments performed was shown in Figure 2.

The experiments were carried out as follows: coformer (compounds) were weighed out in a 1:1 molar ratio and placed in a 1 dram glass vial. Solvent was added to the solid mixture using a calibrated laboratory pipette. For each experiment, 20 microliters of solvent per 100 mg of total solids were added to the top of the solid mixture. The vial was then sealed with a screw cap and then placed in the sample holder. The sample holder was placed into the LABRAM mixer resonant acoustic mixer and secured. The LABRAM mixer was turned on for 2 hours at 90% intensity (approximately 90 times the acceleration of gravity, or 90 g's or g-forces) in auto-resonance mode. The product from the experiment was then analyzed by PXRD over 2-40 degrees, 30 kV, and 15 mA.

Cocrystals can be prepared for a wide variety of compounds including active pharmaceutical ingredients, pharmaceutical intermediates, energetics, nutritionals, and the like. Cocrystal applications may range from, but are not limited to, altering the properties of the cocrystal by the careful selection of cocrystal coformers such as: (1) chemical stability, (2) physical stability, (3) solubility, (4) thermal behavior, (5) impurity inclusion, or rejection within crystallization operations, (6) hygroscopicity, (7) compatibility with formulation components and packaging, (8) to aid in processing, and (9) product performance. That is to say a pharmaceutical cocrystal may contain two or more active pharmaceutical ingredients as coformers. In a similar fashion an energetic cocrystal may contain two or more energetic coformers. In the case where coformer A is an API or energetic, whose properties the cocrystal is intended to modify, it would be typical to screen suitable coformers from a preferred list. The list of coformers is indeed long and the API or energetic compound can theoretically be cocrystallized with either acidic, basic, or neutral coformers in multiple combinations (Wouters, J., Rome, S., and L. Quere, Monograph of most frequent cocrystal formers, Chapter 16, in Fundamental Aspects of Salts and Cocrystals, in Pharmaceutical Salts and Co-cocrystals, Edited by Johan Wouters and Luc Quere, RSC Drug Discovery Publishing 2012, incorporated herein by reference in its entirety). A list of coformers include but are not limited to those in Table 1:

**Table 1: Preferred coformers include but are not limited to:**

| | | |
|---|---|---|
| Acetic acid | Hydrochloric acid | L-pyroglutamic acid |
| o-acetylsalicylic acid | 4-hydroxybenzamide | Resorcinol |
| adipic acid | 4-hydroxybenzoic acid | Saccharin |
| 4-aminobenzoic acid | 1-hydroxy-2-naphthoic acid | Salicylic acid |
| 4-aminobenzamide | Imidazole | Sebacic acid |
| Anthranilic acid | Isonicotinamide | Sodium hydroxide |
| Arabinose | Ketoglutaric acid | Sodium methoxide |
| L-arginine | L-lactamide | Sorbic acid |
| L-ascorbic acid | Lactic acid | Sorbitol |
| L-aspartic acid | Lactose | Stearic acid |
| Benzamide | Laurylsulfonic acid | Suberic acid |
| Benzenesulfonic acid | L-lysine | Succinic acid |
| Benzoic acid | Magnesium chloride | Sucrose |
| Boric acid | Magnesium hydroxide | Tartaric acid |
| Calcium acetate | Maleic acid | L-threonine |
| Calcium chloride | L-Malic acid | Thromethamine |
| Calcium hydroxide | Malonic acid | Trans-cinnamic acid |
| (+) camphoric acid | Maltose | Trimesic acid |
| Camphorsulfonic acid | Mandelic acid | Tyrosine ethyl ester |
| Cholic acid | Mannitol | L-tyrosine |
| Citric acid | Mannose | Urea |
| Cyclamic acid | methanesulfonic | water |
| Ethanol | Methyl-4-hydroxybenzoic acid | Zinc hydroxide |
| Ethanolamine | Neotame | |
| ethylenediamine | Nicotinamide | |
| Ethanesulfonic acid | Nicotinic acid | |
| Erythritol | N-methyl-D-glucamine | |
| Fructose | Orcinol | |
| Fumaric acid | Oxalic acid | |
| Gentisic acid | 2-oxoglutaric acid | |
| Glucose | Palmoic acid | |
| D-glucuronic acid | Pimelic acid | |
| D-gluconic acid | Piperazine | |
| L-glutamic acid | Potassium hydroxide | |
| Glutaric acid | L-proline | |
| Glycine | | |
| Glycolamide | | |
| Glycolic acid | | |
| Hippuric acid | | |

From the list of preferred coformers, the more preferred coformers include but are not limited to:
4,4-bipyridine, nicotinamide, 4-aminobenzoic acid, benzoquinone, terephthalaldehyde, saccharin, 2,6-pyridinecarboxylic acid, aspirin/o-acetylsalicylic acid, anthranilic acid, L-ascorbic acid, cholic acid, citric acid, fumaric acid, glutaric acid, 4-hydroxybenzoic acidmaleic acid, L-malic acid, malonic acid, mandelic acid, oxalic acid, salicylic acid, sorbic acid, stearic acid, succinic acid, tartaric acid, thromethamine, and urea.

Salts of pharmaceutical ingredients or energetic materials are generally more restrictive than cocrystals per se to finding acceptable counter-ions, due to in part to the restrictive stoichiometries of the acid-base pair. Preferred counter ions for pharmaceutical salts include but are not limited to those listed in Table 2:

**Table 2: Preferred counter ions for pharmaceutical salts**

| ***Salt-forming Acids*** | ***Salt-Forming Bases*** |
|---|---|
| Hydrochloride | Sodium |
| Sulfate | Calcium |
| Hydrobromide | Potassium |
| Tartrate | Magnesium |
| Mesylate | Meglumine |
| Maleate | Ammonium |
| Citrate | Aluminum |
| Phosphate | Zinc |
| Acetate | Piperazine |
| Pamoate | Tromethamine |
| Hydroiodide | Lithium |
| Nitrate | Choline |
| Lactate | Diethylamine |
| Methylsulfate | 4-phenylcyclohexylamine |
| Fumarate | Benzathine |

Depending on the relative acidity or basicity of a coformer one may prefer to form a salt with an appropriate counter ion (anion or cation) first and then prepare a cocrystal by combining the aforementioned salt with one or more cocrystal coformers as described above.

A cocrystal may form between one or more coformers plus another molecule that is liquid at room temperature (solvent) or processing temperature and thus may be referred to as the solvate of the cocrystal.

Preferred operation of the LABRAM mixer includes but is not limited to rational stoichiometries such as 0.5:1, 1:1, 1.5:1, and 2:1 mole ratio of coformers.

### EXAMPLES

The present disclosure is demonstrated by the following examples but is not limited to them. To exemplify the process carbamazepine (cbz), an API, was selected as the model compound representing coformer A. Selection of coformers is guided by the functional groups present within a molecular structure which provide the structural building blocks or supramolecular synthons. Coformer B was selected to take advantage of the hydrogen bonding capabilities of carbamazeine. Coformer B can itself be another API, solvent, counterion, salt, or energetic, depending on the application.

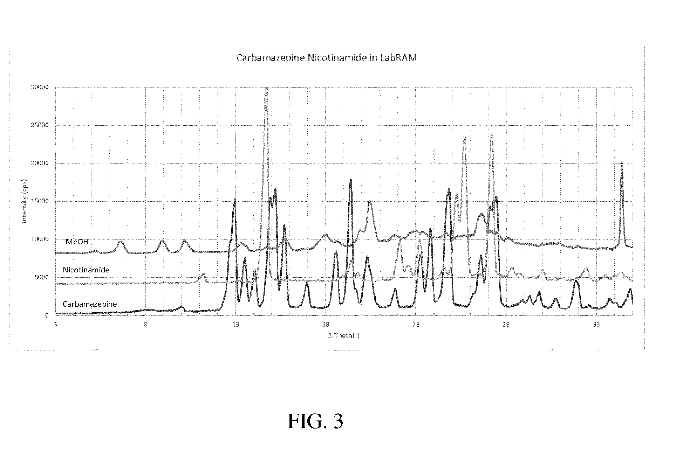

Example 1: 100 mg of coformer A (carbamazepine) and 51.7 mg of coformer B (nicotinamide) were added to a 1 dram vial in a 1:1 stoichiometric ratio. 30 microliters of methanol was added to the vial via pipette. The vial was capped and placed securely into the LABRAM mixer resonant acoustic mixer (Resodyn, Butte, MT). The LABRAM mixer was used to mix the powders for 2 hours at 90% intensity (61 Hz and 100 g-forces) in auto-resonance mode. The powder was analyzed by powder x-ray diffraction (x-ray settings of 40-degrees, 30 Kv, 15 mA), the results of which are shown at Figure 3. Overlaid in Figure 3 as well are the pure component PXRD for carbamazepine and nicotinamide.

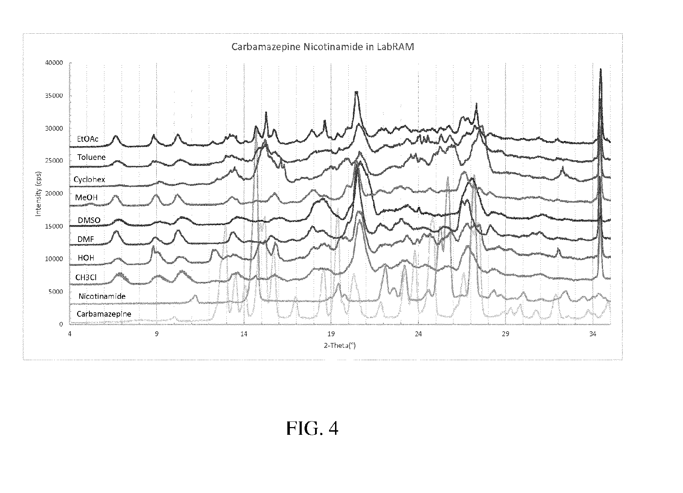

Example 2: Following a similar procedure used in example 1, a series of experiments were performed with carbamazepine and nicotinamide coformers while varying the solvent used during the cocrystallization. 100 mg of carbamazepine and 51.69 mg nicotinamide were mixed with 30 microliters of solvent. Resonant acoustic mixing (LABRAM mixer) was performed at 90% intensity (61 Hz and 100 g-forces). The solvents used were: chloroform (CHCl₃), water, dimethylformamide (DMF), dimethylsulfoxide (DMSO), methanol (MeOH), cyclohexane, toluene, and ethyl acetate. After 2 hours the samples were analyzed by PXRD. The powder x-ray diffraction patterns for this set of 8 experiments are shown in Figure 4. Overlaid in Figure 4 are the pure component PXRD for carbamazepine and nicotinamide. In all cases the carbamazepine:nicotinamide cocrystal was formed, with the exception of cyclohexane where only partial conversion was obtained.

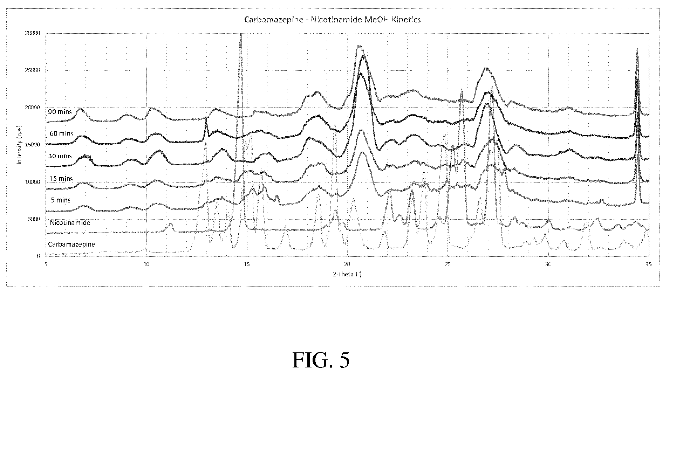

Example 3: 100 mg of carbamazepine and 51.7 mg nicotinamide were added to a 1 dram vial in a 1:1 stoichiometric ratio. 30 microliters of methanol was then added to the vial via pipette. The vial was capped and placed securely into the LABRAM mixer. The LABRAM mixer was used to mix the powders at 90% intensity (61 Hz and 100 g-forces) in auto-resonance mode. The powder was analyzed by PXRD (xray settings of 40-degrees, 30 Kv, 15 mA). Samples were pulled after 5, 15, 30, 60, and 90 minutes of mixing. The data indicated that conversion was rapid with most of the cocrystallization conversion complete after 5 minutes as shown in Figure 5. Overlaid in Figure 5 are the pure component PXRD for carbamazepine and nicotinamide.

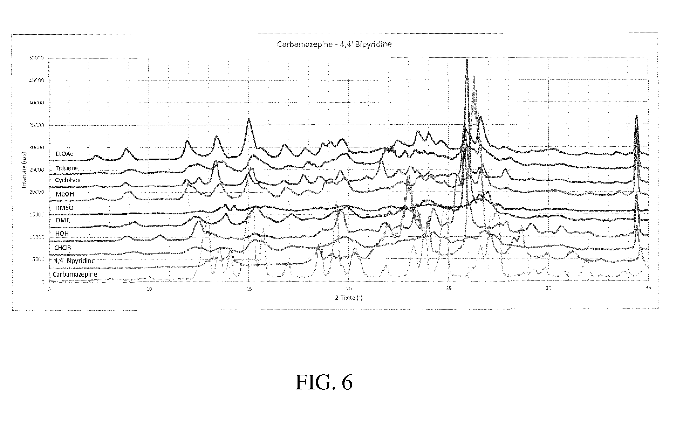

Example 4: A series of experiments were performed with carbamazepine (cbz) and 4,4-bipyridine while varying the solvent used during cocrystallization in the resonant mixer. 100 mg of carbamazepine (cbz) and 66.1 mg of 4,4-bipyridine were mixed (1:1 stoichiometry) with 33 microliters of solvent, and assisted using resonant acoustic mixing (LABRAM mixer) at 90% intensity (61 Hz and 100 g-forces). The individual solvents used were the following: chloroform (CHCl₃), water, dimethylformamide (DMF), dimethylsulfoxide (DMSO), methanol (MeOH), cyclohexane, toluene, and ethyl acetate. Cocrystals were formed in all cases, with the exception of DMSO where only partial conversion was realized or potential amorphous material may have formed. A cocrystal hydrate appears to have been formed from water. The PXRD patterns are shown in Figure 6. Overlaid in Figure 6 are the pure component PXRD for carbamazepine and 4,4-bypyridine.

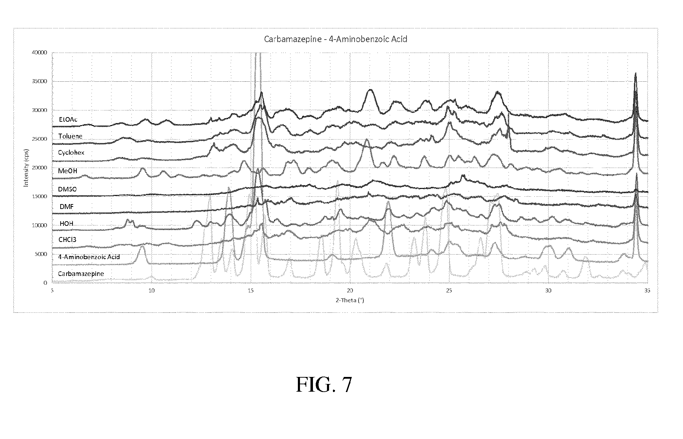

Example 5: A series of experiments were performed with carbamazepine (cbz) and 4-aminobenzoic acid were mixed (1:1 stoichiometry) while varying the solvent used during cocrystallization in the resonant mixer. 100 mg of carbamazepine (cbz) and 58.04 mg 4-aminobenzoic acid were mixed with 32 microliters of solvent, and assisted using resonant acoustic mixing (LABRAM mixer) at 90% intensity (61 Hz and 100 g-forces). The individual solvents used were the following: chloroform (CHCl₃), water, dimethylformamide (DMF), dimethylsulfoxide (DMSO), methanol (MeOH), cyclohexane, toluene, and ethyl acetate. After 2 hours the sample was analyzed by PXRD. The PXRD pattern for this set of 8 experiments are shown in Figure 7. Overlaid in Figure 7 are the pure component PXRD for carbamazepine and 4-aminobenzoic acid. Cocrystals were formed from chloroform, methanol, cyclohexane, and ethyl acetate. A cocrystal hydrate was formed from water. Little or no conversion appeared to have occurred with DMF. From DMSO and toluene only partial conversion or conversion to amorphous solid was obtained.

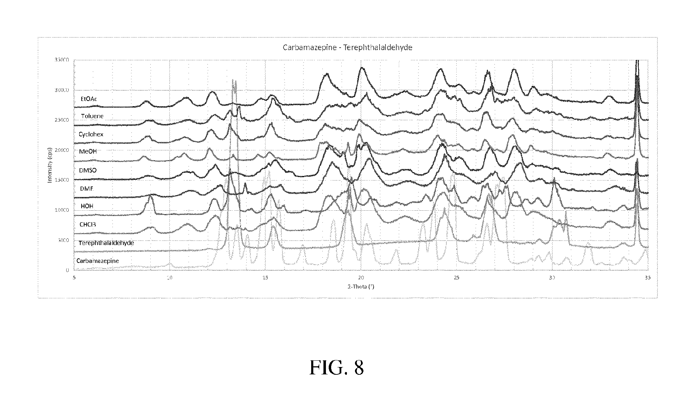

Example 6: Following the same procedure as example 2, a series of experiments were performed with carbamazepine (cbz) and terephthaladehyde wherein each were mixed to (1:1 stoichiometry) while varying the solvent used during cocrystallization in the resonant-acoustic mixer. 100 mg of carbamazepine (cbz) and 56.8 mg terephthalaldehyde were mixed with 31 microliters of solvent, and assisted using resonant acoustic mixing (LABRAM mixer) at 90% intensity (61 Hz and 100 g-forces). The individual solvents used were the following: chloroform (CHCl₃), water, dimethylformamide (DMF), dimethylsulfoxide (DMSO), methanol (MeOH), cyclohexane, toluene, and ethyl acetate. After 2 hours the sample was analyzed by PXRD. The PXRD pattern for this set of 8 experiments are shown in Figure 8. Overlaid in Figure 8 are the pure component PXRD for carbamazepine and terephtlalaldehyde. Cocrystals were formed in all cases.

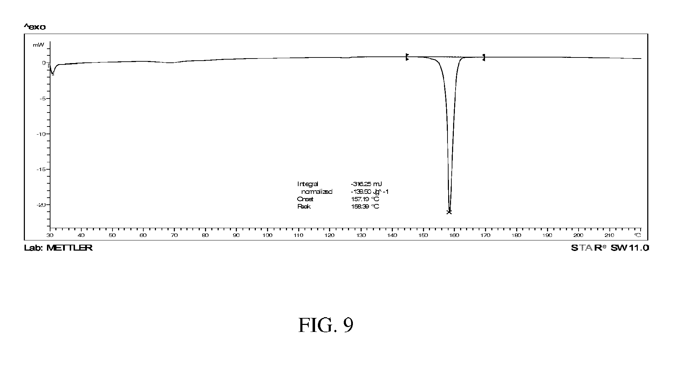

Example 7: Small scale screening of cocrystals using LABRAM mixer was performed. A cocrystal of carbamazepine:nicotinamide was isolated using 20 microliters of deionized (DI) water per 100 mg of solids in the LABRAM mixer. The DSC of carbamazepine:nicotinamide cocrystal is shown in Figure 9 with melting point (onset) of 157.2 °C and heat of fusion of 139.5 J/g uncorrected residual for solvent content. The peak of the endotherm is at 158.4 °C.

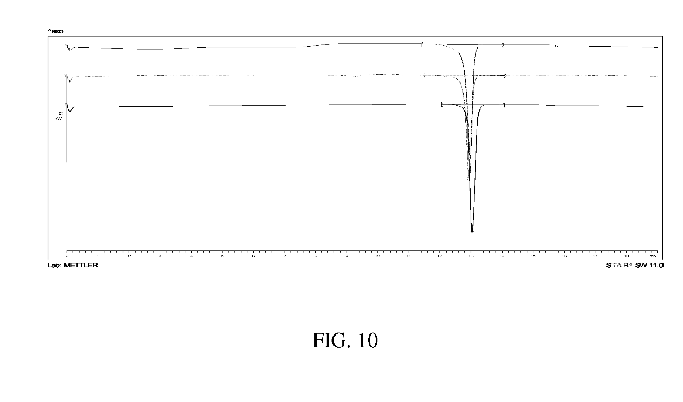

Example 8: Isolation procedure for increased crystallinity and purity: 100 mg carbamazepine and 52 mg nicotinamide were cocrystallized in the presence of 30 microliters of dimethylformamide (DMF). The experiment was stopped after one hour at 90% intensity in the LABRAM mixer. The cocrystals were hard-packed in the bottom of the vial; DSC indicated excellent conversion based on the single endotherm and melting point of 157.3°C. The solids were dried overnight at 70°C and 28 in Hg of vacuum. A DSC of the dried solids indicated a heat of fusion of 140 J/g and melting point onset of 157.5°C. The dry solids were then reslurried in the LABRAM mixer with 6 volumes of acetonitrile at 30% intensity for 5 minutes resulting in a fine slurry. The solids were filtered and dried at 65°C and 28 in Hg of vacuum overnight. The DSC curves are shown in Figure 10. The three DSC curves in Figure 10 show carbamazepine:nicotinamide a) wet with DMF, b) after drying of residual DMF, c) after reslurry in acetonitrile, filtration, and drying of residual acetonitrile. Melting point and heat of fusion increases with purity. The heat of fusion for the initial wet cake after 1 hour of resonant acoustic mixing was 123 J/g (DMF wet) and after drying was 138 J/g. The heat of fusion of the reslurried (acetonitrile), filtered, and dried material was 154 J/g.

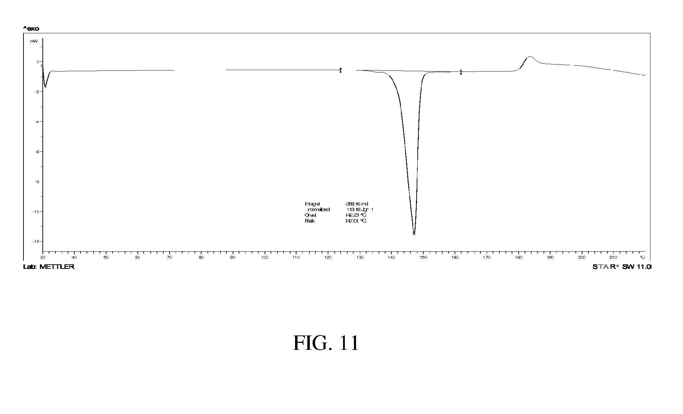

Example 9: 4-aminobenzoic acid was selected as a coformer with carbamazepine. In the presence of 32 microliters of methanol a 1:1 carbamazepine:4-aminobenzoic acid cocrystal was produced during resonance acoustic mixing with MeOH. The product formed as a blend of agglomerates/granules and powder. For the powder the melting onset was 142°C with a peak at 147°C and a heat of fusion of 113.8 J/g based on DSC as is shown in Figure 11.

Example 10: Scale-up experiments using resonant acoustic mixing for preparing cocrystals:
1.0075 g of carbamazepine and 0.516 g of nicotinamide were combined in a vial. 304 microliters (20 microliter/100 mg of solids) of DI water was added. The wet cake was mixed for 1 hour at 59 g's (62 Hz). A blend of powder and granules was formed. DSC indicated a small amount of nicotinamide still present so an additional hour of mixing was provided. The granules were separated from the powder and tested separately. The heat of fusion for the wet agglomerated granules was 130 J/g with a melting onset of 156.6°C. The cocrystal powder had a heat of fusion of 148.5 J/g with a melting point of 156.7°C. Thus 1.5 g of carbamazepine:nicotinamide cocrystals were generated in the form of granules and powder when water was used.

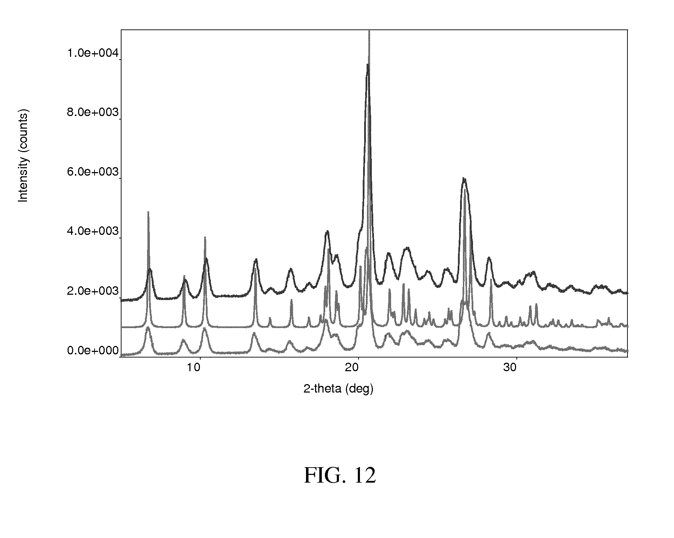

In a separate experiment 1.00 g of carbamazepine and 0.517 g of nicotinamide were combined in a vial; the solids were premixed at 30% intensity for 5 minutes followed by the subsequent addition of 300 microliters of dimethylformamide (DMF) to the solid mixture. The cocrystal wet cake was caked to the bottom of the vessel after running the LABRAM mixer for one hour at 80% intensity in auto resonance mode. Six volumes of acetonitrile was added to the wet cake. The slurry was agitated for approximately one minute at 20% intensity to reslurry the solids. Only a mild intensity was required to reslurry and suspend the solids. The slurry was then filtered and the solids dried on a watch-glass overnight at 50°C and 28 in Hg of vacuum. The PXRD pattern of the isolated solids is shown in Figure 12. DSC of the dried cocrystal showed ΔH_{fusion} of 140 J/g at 158.4°C melting onset and 159.2 °C peak temperature.

Carbamazepine:nicotinamide (1:1) in DMF process was further scaled to 22.75 g in the resonance acoustic mixer. In this experiment a 300-ml glass jar was used with a secondary plastic container. 15.0 grams of carbamazepine were weighed into a sample jar. Next, nicotinamide was sieved through a 500 micron sieve to break-up any hard lumps and 7.75 g of the sieved material was added to the sample jar providing a 1:1 molar ratio. The dry powder blend was mixed in the LABRAM mixer for 5 minutes at 30% intensity to mix the powders before solvent addition. The jar was opened, and to it was added 4.55 ml (4.82 g) of DMF solvent. The solvent was poured on top of the solids from a beaker. The resonance mixing was set for 80% intensity for one hour. After one hour the reaction was sampled for analysis by PXRD, confirming that the cocrystal product was formed. Figure 12 shows a PXRD overlay of carbamazepine:nicotinamide cocrystal prepared from DMF with acetonitrile reslurry on a 1.5 gram scale and 22 gram scale by resonance acoustic mixing compared with a calculated powder pattern from single crystal structure for carbamazepine:nicotinamide (I) obtained from Fleischman et. al. (Fleischman, S. G., Kuduva, S. S., McMahon, J. A., Moulton, B., Bailey, R. D., Walsh, Rodríguez-Hornedo, N., Zaworotko, M. J., Crystal Growth & Design, 2003, 3, 6, 909-919, incorporated herein by reference in its entirety).

During resonance the wet-cake appears to be "fluidized" as the solids and solvent were intimately mixed until eventually the wet solids become caked to the bottom of the vessel. Five volumes (115 ml) of acetonitrile were then added for the reslurry step. The sample container was placed back into the LABRAM mixer to reslurry the solids at 20% intensity for 5 minutes. The slurry was then transferred to a glass Buchner funnel and one volume (24 ml) of acetonitrile was used to rinse solids from the original sample container. The solids were transferred to the filter; the solids filtered fast, the filtrate was clear, and the solids were dried at 50°C and 27 in Hg of vacuum overnight. The isolated yield of 86% includes physical losses and losses in filtrate. Some cake hardening was observed upon drying requiring that it be delumped through a 500 micron sieve. A DSC of the cocrystal product showed a ΔH_{fusion} of 141 J/g at 158.9°C (melting onset). Cocrystals prepared by the solution cooling crystallization and solvent evaporation methods as reported in literature showed melting peaks at 160°C with a heat of fusion 150.4 J/g (Ziyaur Rahman, Cyrus Agarabi Ahmed S. Zidan, Saeed R. Khan, and Mansoor A. Khan, Physico-mechanical and Stability Evaluation of Carbamazepine Cocrystal with Nicotinamide AAPS PharmSciTech, Vol. 12, No. 2, June 2011 (# 2011), incorporated by reference herein in its entirety). The PXRD pattern for the cocrystal produced at the 22 gram scale is shown in Figure 12.

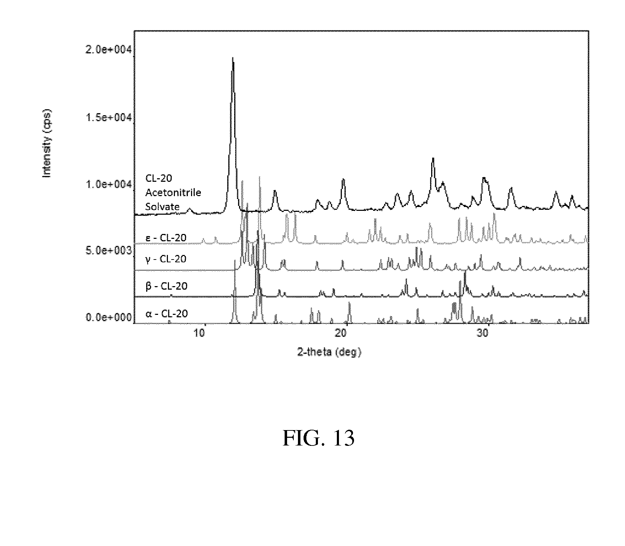

Example 11: Hexanitrohexaazaisowurtzitane (CL20) is known to exhibit multiple polymorphs. 50 mg of CL20 (epsilon form) was charged to the LABRAM mixer vial with 20 microliter of (3:1) acetonitrile/2-propanol; after 60 minutes and 80 % intensity in LABRAM mixer, the sample was removed. PXRD indicated a distinct form as shown in Figure 13. Figure 13 is overlaid with known polymorphic forms of CL20 as well. The PXRD was found to be consistent with the calculated powder pattern from single crystal of a CL20-acetonitrile-solvate. Thus LABRAM mixer was used to generate a CL20-acetonitrile-solvate form directly from the epsilon form of CL-20.

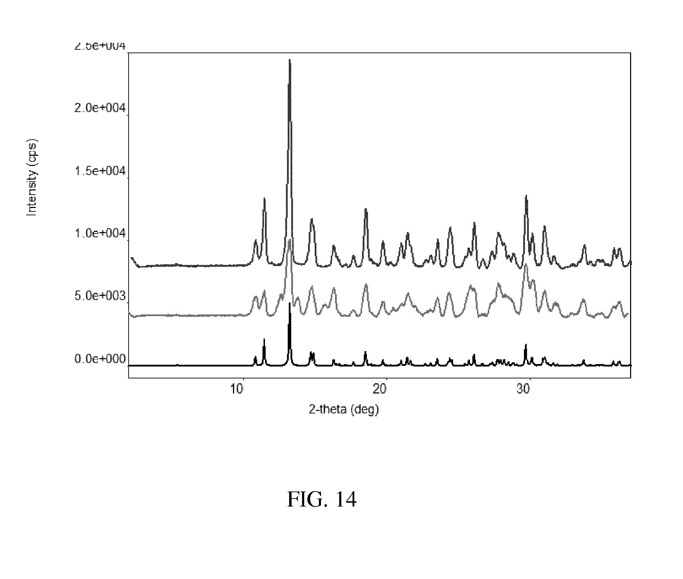

Example 12: Energetic-energetic cocrystals produced by resonant acoustic mixing is exemplified using CL-20 and HMX. A 2:1 molar ratio cocrystal consisting of Hexanitrohexaazaisowurtzitane (CL20) and octahydro-1,3,5,7-tetranitro-1.3,5,7-tetrazocine (HMX) respectively, was prepared using RAM. 80 mg of CL20 was combined with 27 mg of HMX in a small container. 33 microliters of a 30 wt% acetonitrile in 2-propanol solution was added to the solid mixture. The mixture was placed in the LABRAM and mixed at 80 g-forces for 1 hr. This method resulted in high yield approximately 100% cocrystal. The powder diffraction pattern of cocrystal obtained through RAM matches the 2CL20:HMX cocrystal prepared from solution as shown in Figure 14.

These experiments clearly indicate that resonant acoustic mixing facilitates the formation of cocrystals, polymorphs, and solvates.

Highly uniform and high intensity mixing promoting solid-solid particle collisions enables the effective cocrystallization process. The process is significantly faster when assisted by the presence of solvent. In this way, the process is solvent mediated at the solid-liquid interface while the highly uniform and intense mixing provides the energy that is distributed uniformly through the mixture to afford facile conversion to cocrystalline product. Mixers that can provide resonant energy intensity of 10 to 1000 g-forces and preferably in the vicinity of 100 g-forces with mixing frequency of 15 to 1000 Hz with an amplitude, on the order of but not limited to, between 0.01 to 0.5 inches are capable of synthesis of cocrystals.

One such system, for use in a preferred embodiment, is manufactured by Resodyn Acoustic Mixers, Inc. (Butte, Montana) and is disclosed in US Patent 7,188,993 B1, which is incorporated herein by reference in its entirety. The '993 patent describes a system that is capable of achieving frequency of mixing in the range 10 to 1000 Hz, accelerations of 2-100 times the force of gravity (g's), and displacement amplitude of 0.01 to 0.5 inches.

Cocrystallization of coformers can be mixed with varying stoichiometric ratios. In some cases 1:1 stoichiometric ratios are preferred while 2:1 or 1:2 ratios are also common. Other ratios are possible depending on the hydrogen bonding sites within the respective coformers. Salt forms may have 1:1 stoichiometry but are not limited to 1:1 stoichiometry. Similarly hydrates and solvates are not limited to 1:1 stoichiometry.

Although the disclosed subject matter has been described and illustrated with respect to embodiments thereof, it should be understood by those skilled in the art that features of the disclosed embodiments can be combined, rearranged, etc., to produce additional embodiments within the scope of the invention, and that various other changes, omissions, and additions may be made therein and thereto, without parting from the spirit and scope of the present disclosure. Variations in mode of solvent addition, solvent concentration and loading, temperatures, including the use of adding grinding media to the cocrystal mixture are considered within scope.

## Claims

1. A process for making a cocrystal from two or more compounds by combining compounds in a suitable vessel and subjecting the vessel and mixture to resonant acoustic mixing.

2. A process for making a crystalline salt form by combining two compounds in a suitable vessel and subjecting the vessel and mixture to resonant acoustic mixing.

3. The process according to claim 1 wherein a solvent or mixture of solvents is added or the presence of water vapor leads to the formation of a cocrystal hydrate or cocrystal solvate.

4. The process according to any one of claims 1 to 3 wherein a resonant frequency of mixing is from about 10 Hz to about 2000 Hz and acceleration energies for mixing in the range of 2 and 200 g-forces.

5. The process according to claim 4 wherein the resonant frequency of mixing is from about 50 to 100 Hz with resultant accelerations for mixing in the range of about 10 and 150 g-forces.

6. The process of claim 4 where the intensity of mixing is between about 10 to 150 g-forces.

7. The process according to claim 1 or 2 wherein a solvent or mixture of solvents is added.

8. A process according to claim 1 further comprising increasing a volume of the resonant acoustic mixer to perform a scaled-up process for making a cocrystal from two or more compounds.

9. The process of claim 1, wherein one of the compounds is one of a salt, a neutral non-ionized compound, an energetic compound, or an active pharmaceutical ingredient.

10. The process according to claim 2 wherein one of the compounds is one of a pharmaceutical compound, an energetic compound, or a suitable counter-ion.

11. The process according to claim 1 wherein at least one of the compounds is a coformer.

12. The process according to claim 11, wherein a solvent is added in which one or more coformers is slightly soluble to mediate the solid-solid interactions between the coformers.

13. The process according to claim 2 or 3 wherein a scaled-up preparation of a salt formation, solvate, or polymorph using resonant acoustic mixing is performed.

14. A process for effecting a polymorph transformation of a compound by combining the compound in the presence of a solvent or mixture of solvents in a suitable vessel and subjecting the vessel and the mixture to resonant acoustic mixing.

15. A process for screening solid state forms such as cocrystals, salts, and polymorphs with the intention of discovering new solid state forms of cocrystals or salts, or alternatively acid, base, or neutral components of cocrystals or salts including varying experimental conditions using resonant acoustic mixing.

16. The process according to claim 1 wherein an o-acetylsalicyclic acid product or a carbamazepine product is obtained.
